(19) Europäisches Patentamt
European Patent Office
Office européen des brevets

(11) Veröffentlichungsnummer: **0 383 239**
**A1**

# (12) EUROPÄISCHE PATENTANMELDUNG

(21) Anmeldenummer: **90102731.8**

(22) Anmeldetag: **12.02.90**

(51) Int. Cl.[5]: **C07D 473/26, C07D 307/20, C07D 307/22, C07D 339/08, C07D 405/12, C07D 409/04, A61K 31/52**

(30) Priorität: **13.02.89 US 309561**

(43) Veröffentlichungstag der Anmeldung: **22.08.90 Patentblatt 90/34**

(84) Benannte Vertragsstaaten: **AT BE CH DE DK ES FR GB GR IT LI LU NL SE**

(71) Anmelder: **F. HOFFMANN-LA ROCHE AG**
**Postfach 3255**
**CH-4002 Basel(CH)**

(72) Erfinder: **Huryn, Donna Mary**
**164 Hillcroft Way**
**Newton, Pennsylvania 18940(US)**
Erfinder: **Tam, Steve Yik-Kai**
**13 Evergreen Road**
**West Caldwell, N.J. 07006(US)**
Erfinder: **Weigele, Manfred**
**40 Hooyman Drive**
**Clifton, N.J. 07013(US)**

(74) Vertreter: **Lederer, Franz, Dr. et al**
**Lederer, Keller & Riederer, Patentanwälte,**
**Lucile-Grahn-Strasse 22**
**D-8000 München 80(DE)**

## (54) Isomere Dideoxynucleoside, ihre Herstellung und Verwendung für Heilmittel.

(57) Die Verbindungen der Formel

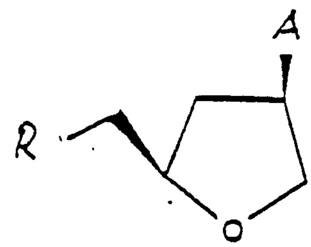

- I

worin A 6-Amino-9H-purin-9-yl oder 2-Amino-6-hydroxy-9H-purin-9-yl darstellt, worin die Aminogruppe durch NHC(O)$R^2$ oder N = CH$R^3R^4$ ersetzt sein kann und R OH oder OC(O)$R^1$ ist, wobei $R^1$, $R^2$, $R^3$ und $R^4$ unabhängig voneinander geradkettiges oder verzweigtes $C_{1-20}$-Alkyl, $C_{1-12}$-Alkoxyalkyl, substituiertes oder unsubstituiertes Phenoxy-$C_{1-4}$-alkyl, Phenyl oder Phenyl das durch Halogen, $C_{1-4}$-Alkoxy, Nitro, Hydroxy, Carboxyl oder Alkylamino substituiert ist, bedeuten und pharmazeutisch anwendbare Salze davon sind als Mittel zur Behandlung retroviraler Infektionen, wie HIV, von Wert.

EP 0 383 239 A1

# Isomere Dideoxynucleoside, ihre Herstellung und Verwendung für Heilmittel

Die vorliegende Erfindung betrifft 2′,3′-Dideoxynucleosid-Isomere der Formel

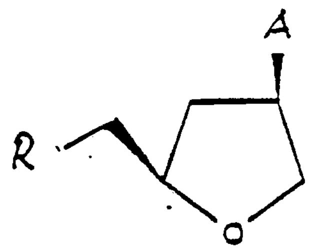

I

worin A 6-Amino-9H-purin-9-yl oder 2-Amino-6-hydroxy-9H-purin-9-yl darstellt, worin die Aminogruppe durch $NHC(O)R^2$ oder $N=CHR^3R^4$ ersetzt sein kann und R OH oder $OC(O)R^1$ ist, wobei $R^1$, $R^2$, $R^3$ und $R^4$ unabhängig voneinander geradkettiges oder verzweigtes $C_{1-20}$-Alkyl, $C_{1-12}$-Alkoxyalkyl, substituiertes oder unsubstituiertes Phenoxy-$C_{1-4}$-alkyl, Phenyl oder Phenyl das durch Halogen, $C_{1-4}$-Alkoxy, Nitro, Hydroxy, Carboxyl oder Alkylamino substituiert ist, bedeuten und pharmazeutisch anwendbare Salze davon; sowie pharmazeutische Präparate, die diese Verbindungen enthalten.

Die vorliegende Erfindung betrifft weiterhin ein Verfahren zur Herstellung der erfindungsgemässen Verbindungen sowie darin verwendete neue Zwischenprodukte.

Die vorliegende Erfindung betrifft weiterhin die Verwendung der erfindungsgemässen Verbindungen zur Herstellung von pharmazeutischen Präparaten zur Behandlung retroviraler Infektionen, insbesondere HIV.

Der hier verwendete Ausdruck Niederalkyl bedeutet Alkyl mit 1-7 C-Atomen. Halogen bedeutet Fluor, Chlor, Brom und Jod. Der Ausdruck Nucleobase bezieht sich auf in Nucleosiden wie Adenin, Guanin, Cytosin, Thymin, 2,6-Diaminopurin, Uracil, Purin oder 2-Aminopurin anwesende Basen. AIBN bedeutet einen üblichen Radikalinitiator wie $\alpha,\alpha'$-Azaisobutyronitril. Eine Abgangsgruppe bedeutet eine funktionelle Gruppe die für nukleophile Verdrängungsreaktionen geeignet ist, wie Alkyl- oder Arylsulfonate, aktivierte OH-Gruppen wie $OP^{(+)}(Phenyl)_3$ oder Halogene. 18-Krone-6 bedeutet ein übliches Phasentransferreagens wie 1, 4, 7, 10, 13, 16-Hexaoxacyclooctadecan. Eine sich verdickende Linie bedeutet, dass der Rest oberhalb der Molekülebene liegt. Eine unterbrochene oder gestrichelte Linie bedeutet, dass der Rest unterhalb der Molekülebene liegt. Eine Wellenlinie bedeutet, dass der Rest oberhalb oder unterhalb der Molekülebene liegen kann. Der Ausdruck "maskierter Vorläufer" bedeutet eine Verbindung, in der eine spezifische funktionelle Gruppe in einer Form vorliegt, die durch einfache Umwandlungen leicht in diese funktionelle Gruppe übergeführt werden kann. Beispielsweise sind Acetale und Ester maskierte Vorläufer von Hydroxymethylgruppen, Aryläther und Silyläther sind maskierte Vorläufer von Hydroxygruppen.

Bevorzugte Verbindungen der Formel I sind diejenigen, in denen A unsubstituiertes 6-Amino-9H-purin-9-yl oder unsubstituiertes 2-Amino-6-hydroxy-9H-purin-9-yl darstellt, insbesondere die Verbindungen Iso-dideoxyadenosin (Ia, IsoddA) und Iso-dideoxyguanosin (Ib, IsoddG) und deren pharmazeutisch anwendbare Salze sowie pharmazeutische Präparate, die diese Verbindungen enthalten. Die pharmazeutisch anwendbaren Salze können Salze organischer Säuren, wie Milchsäure, Essigsäure, Aepfelsäure oder p-Toluolsulfonsäure sein, wie auch Salze pharmazeutisch anwendbarer Mineralsäuren, wie Salzsäure oder Schwefelsäure.

Die Verbindungen der Formel I können erfindungsgemäss dadurch hergestellt werden, dass man

a) eine Verbindung der Formel

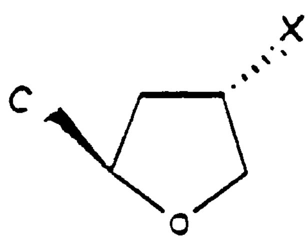

IV-1

worin X eine abspaltbare Gruppe und C Hydroxymethyl oder ein maskierter Vorläufer davon ist, mit Adenin oder Guanin umsetzt und erforderlichenfalls einen maskierten Vorläufer C in eine Hydroxymethylgruppe

überführt oder
b) den Chlorsubstituenten in einer Verbindung der Formel

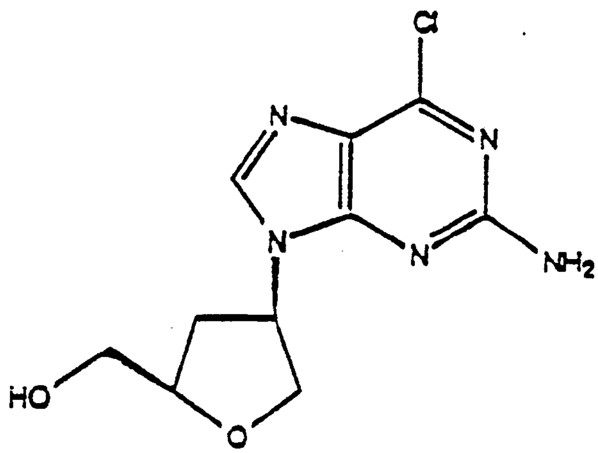

VIII

hydrolysiert oder
c) eine Verbindung der Formel

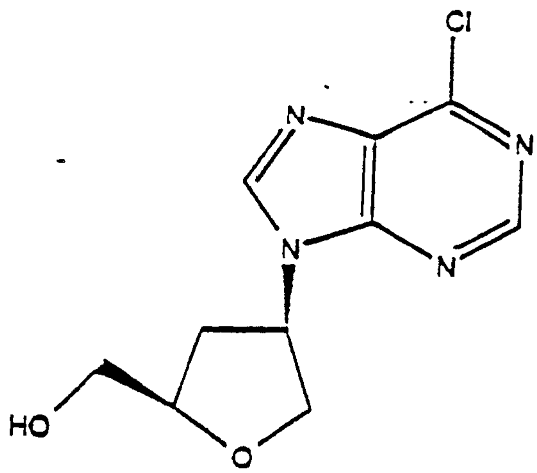

X

mit Ammoniak umsetzt oder
d) eine Verbindung der Formel

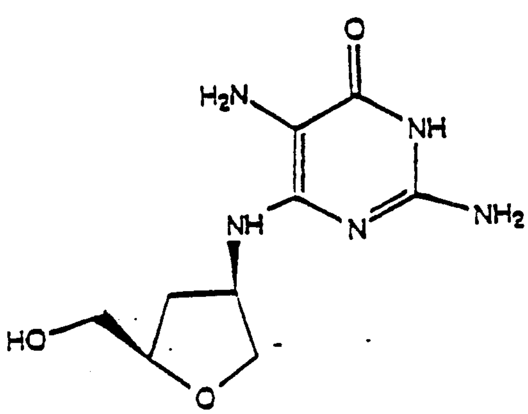

XIII

mit einem Orthoameisensäureester oder mit Diäthoxymethylacetat umsetzt
und gewünschtenfalls die erhaltene Verbindung der Formel I in ein pharmazeutisch anwendbares Salz oder ein N- oder O-Acyl-Derivat oder eine Schiff'sche Base umwandelt.

Die Herstellung der Verbindungen der Formel I und der neuen Zwischenprodukte ist nachstehend detaillierter beschrieben.

Bei der Herstellung der erfindungsgemässen Verbindungen kann man mit einem einfachen Zuckerderivat wie 1,2-0-Methyläthyliden-D-xylofuranose beginnen, das nach einer Reihe von Reaktionen in ein Tosylacetonid-Zwischenprodukt nach dem folgenden Reaktionsschema umgewandelt wird:

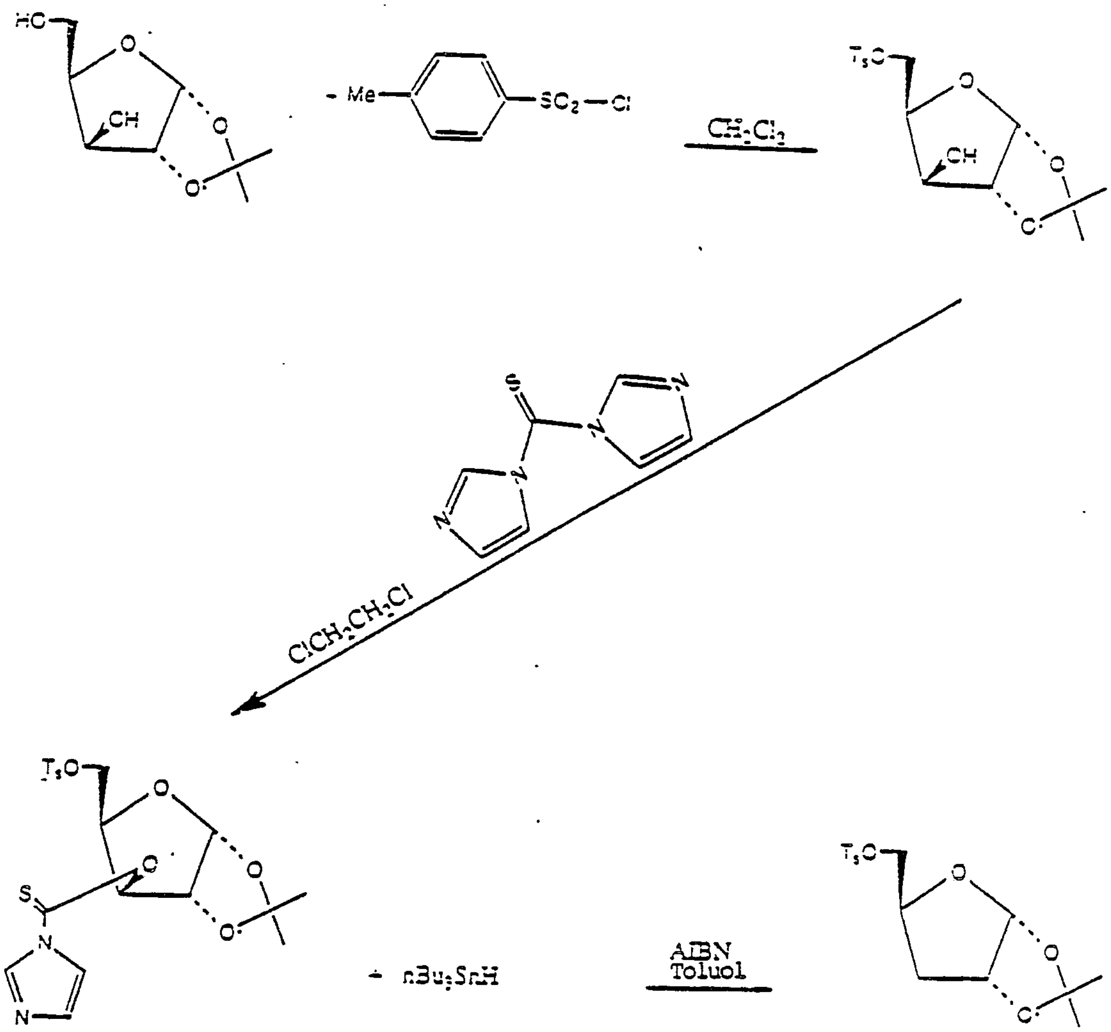


III

Im obigen Reationsschema wird 1,2-0-(1-Methyläthyliden)-α-D-xylofuranose mit Tosylchlorid zu 1,2-0-(1-Methyläthyliden)-5-0-p-(tolylsulfonyl) -α-D-xylofuranose umgesetzt. Die erhaltene Verbindung wird dann mit 1,2-Dichloräthan und Thiocarbonyldiimidazol umgesetzt wobei ein neues Imidazolcarbothiosäurederivat (Verbindung III) erhalten wird, was nach Behandlung mit tri-n-Butylzinnhydrid und AIBN in Toluol 3-Deoxy-1,2-0-(1-methyläthyliden)-α-D-erythro-pentofuranose-4-methylbenzolsulfonat liefert. Dieses Tosylacetonid kann dann auf zweierlei Weise in ein neues Zwischenprodukt der Formel

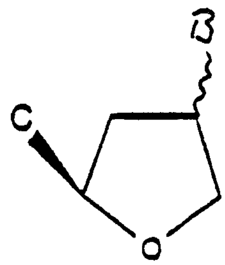

IV

umgewandelt werden, wobei B $NH_2$, Hydroxy oder eine abgewandelte Form davon oder deren maskierte Vorläufer oder eine Abgangsgruppe bedeuten und C Hydroxymethyl oder einen maskierten Vorläufer davon wie Halomethyl, Carbonsäure, Aldehyde, Ester, Acetale, Thioacetale oder Alkoxymethyl darstellen. Das Zwischenprodukt dient dann als Basis für die Synthese einer breiten Palette von Dideoxynucleosidisomeren einschliesslich der Verbindungen Ia und Ib.

Das neue Zwischenprodukt kann nach dem folgenden Schema hergestellt werden

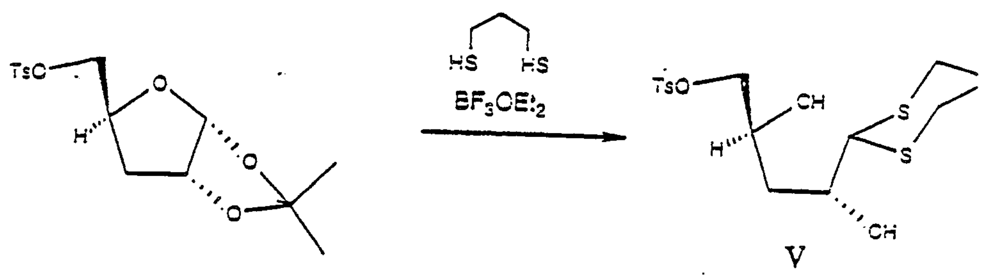


V

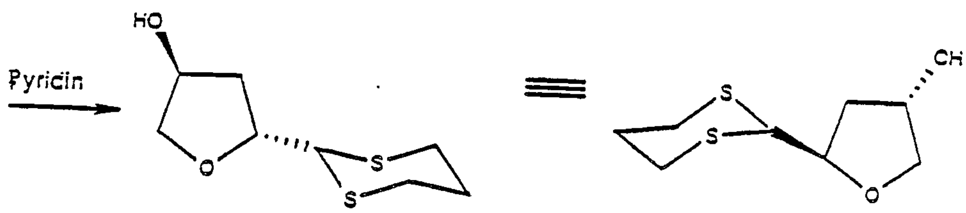


IV (a)

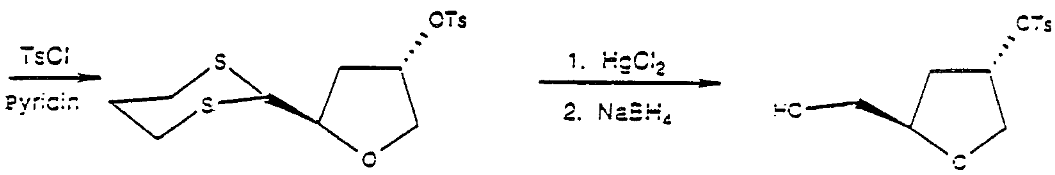


IV (b)

IV (c)

Das Tosylacetonid wird mit 1,2-Propandithiol und Bortrifluoridätherat umgesetzt wobei das offenkettige Diol erhalten wird. Nach Reinigung des offenkettigen Diols V wird dieses in einer Lösung von Methylenchlorid und Pyridin gerührt, wobei das Tetrahydrofuran IV(a) erhalten wird, das unmittelbar mit Tosylchlorid zum tosylierten Thioacetal IV(b) umgesetzt wird. Nach Reinigung des tosylierten Thioacetals durch Säulenchromatographie folgt die Umwandlung in das neue Zwischenprodukt IV(c) durch Hydrolyse mit Quecksilberchlorid gefolgt von Reaktion mit Natriumborhydrid. In diesem Reaktionsschema sind die Verbindungen IV-(a), (b), (c) und V neue Zwischenprodukte.

Die Verbindung IV kann auch nach dem folgenden Schema hergestellt werden

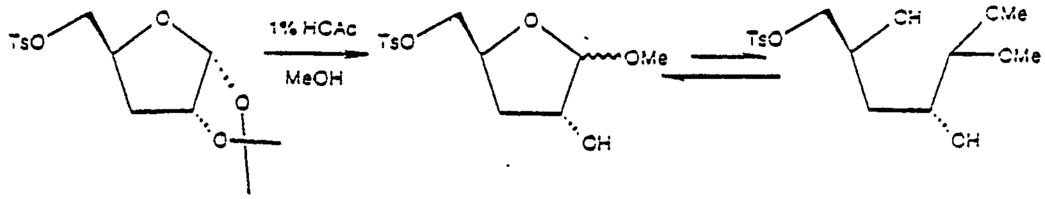


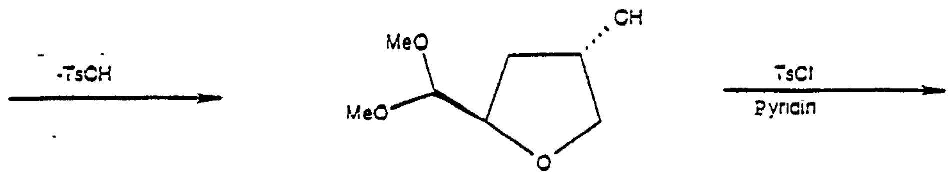


IV (d)

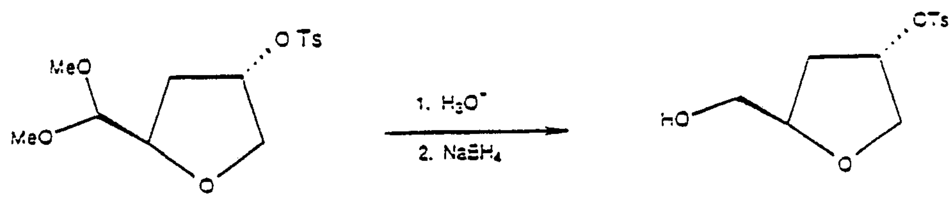


IV (e)

IV (c)

In diesem Reaktionsschema wird das Tosylacetonid mit Essigsäure/Methanol zu einem Gemisch des Methylglycosids, des ringgeöffneten Dimethylacetals und des gewünschten Produkts IV(d) umgesetzt. Nach Rühren dieses Gemisches während mehrerer Tage wird die vollständige Umwandlung zum Dimethylacetaltetrahydrofuran IV(d) erreicht. Diese ungereinigte Verbindung wird dann unter üblichen Bedingungen tosyliert, wobei das Dimethylacetaltosylat IV(e) erhalten wird, das dann in die Verbindung IV(c) durch Säurehydrolyse und Natriumborhydridreduktion umgewandelt wird. In diesem Schema sind die Verbindungen IV(d) und IV(e) neue Zwischenprodukte.

Die Verbindung IV kann zur Herstellung einer Vielzahl von Nukleosiden einschliesslich der Verbindung Ia und Ib nach zwei grundsätzlichen Strategien verwendet werden.

Die erste Möglichkeit besteht in einer direkten Verdrängung der Abgangsgruppe am Isodideoxyzucker durch eine Nukleobase wie in Gleichung 1 dargestellt. Die Synthese carbocyclischer Analoge von Nukleosiden (insbesondere von Adeninderivaten) nach diesem Reaktionstyp ist in der Literatur beschrieben worden, die Methode scheint jedoch nicht viel verwendet zu werden.

Gleichung 1:

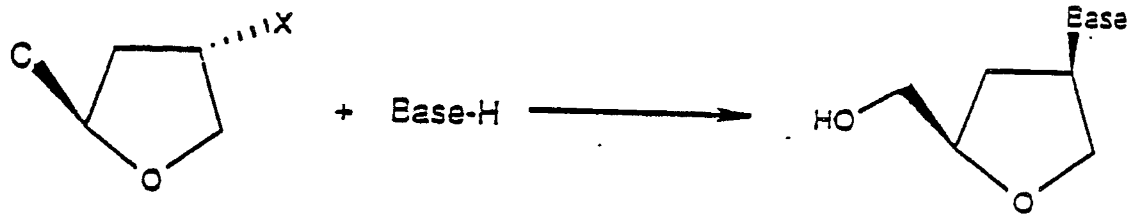

X = Abgangsgruppe

Base-H = Nukleobase

C = $CH_2OH$ oder dessen maskierter Vorläufer

Die alternative klassische Methode beruht auf dem schrittweisen Aufbau der Nukleobase. Die Synthese von Nukleosiden, insbesondere Purin- und Pyrimidinderivaten nach dieser Verfahrensweise ist in der Literatur gut belegt. Es ist zu erwarten, dass alle klassischen Nukleobasen in die Isodideoxynukleosidstruktur ausgehend vom Isodideoxyzucker IV eingebaut werden können. Das Aminoderivat IV(f) ist aus IV durch Standardumwandlungen leicht erhältlich, so dass das gleiche Zwischenprodukt für beide Verfahrensweisen verwendet werden kann. In Gleichung 2 stellt die Verbindung IV(f) ein neues Zwischenprodukt dar.

Gleichung 2:

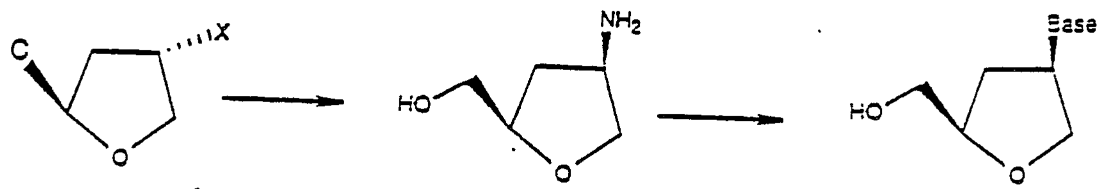

IV - 1 IV (f)

X = Abgangsgruppe

C = $CH_2OH$ oder dessen maskierter Vorläufer

Beispielsweise kann die Verbindung Ia, IsoddA, dadurch synthetisiert werden, dass man die Verbindung IV(c) entweder mit Adenin, Kaliumcarbonat und einem Phasentransferreagens, wie 18-Krone-6 oder mit dem Natriumsalz von Adenin wie nachstehend dargestellt, umsetzt.

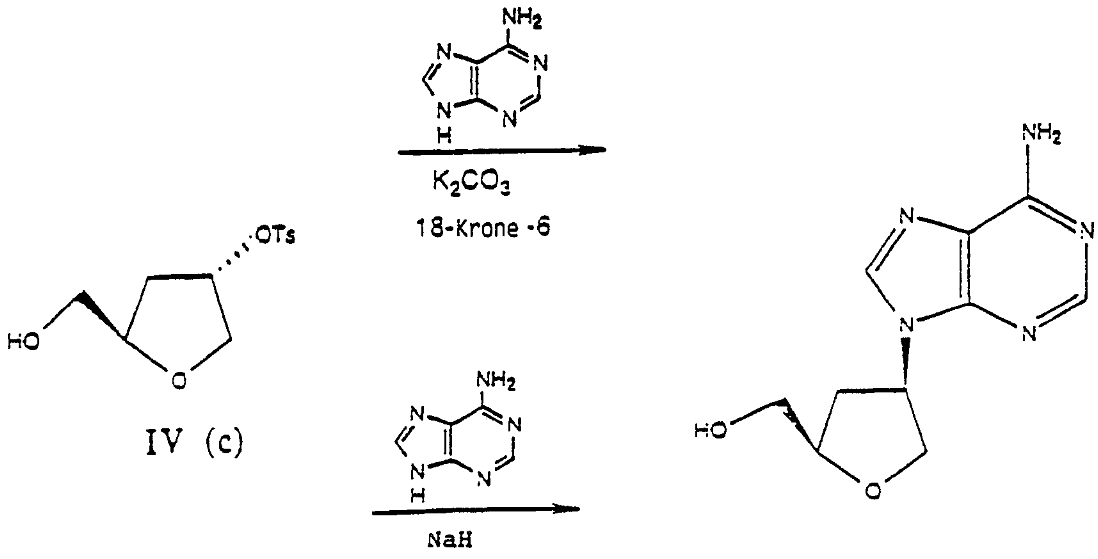


Die Verbindung IV(e) kann zur Herstellung der Verbindung I nach folgendem Schema verwendet werden:

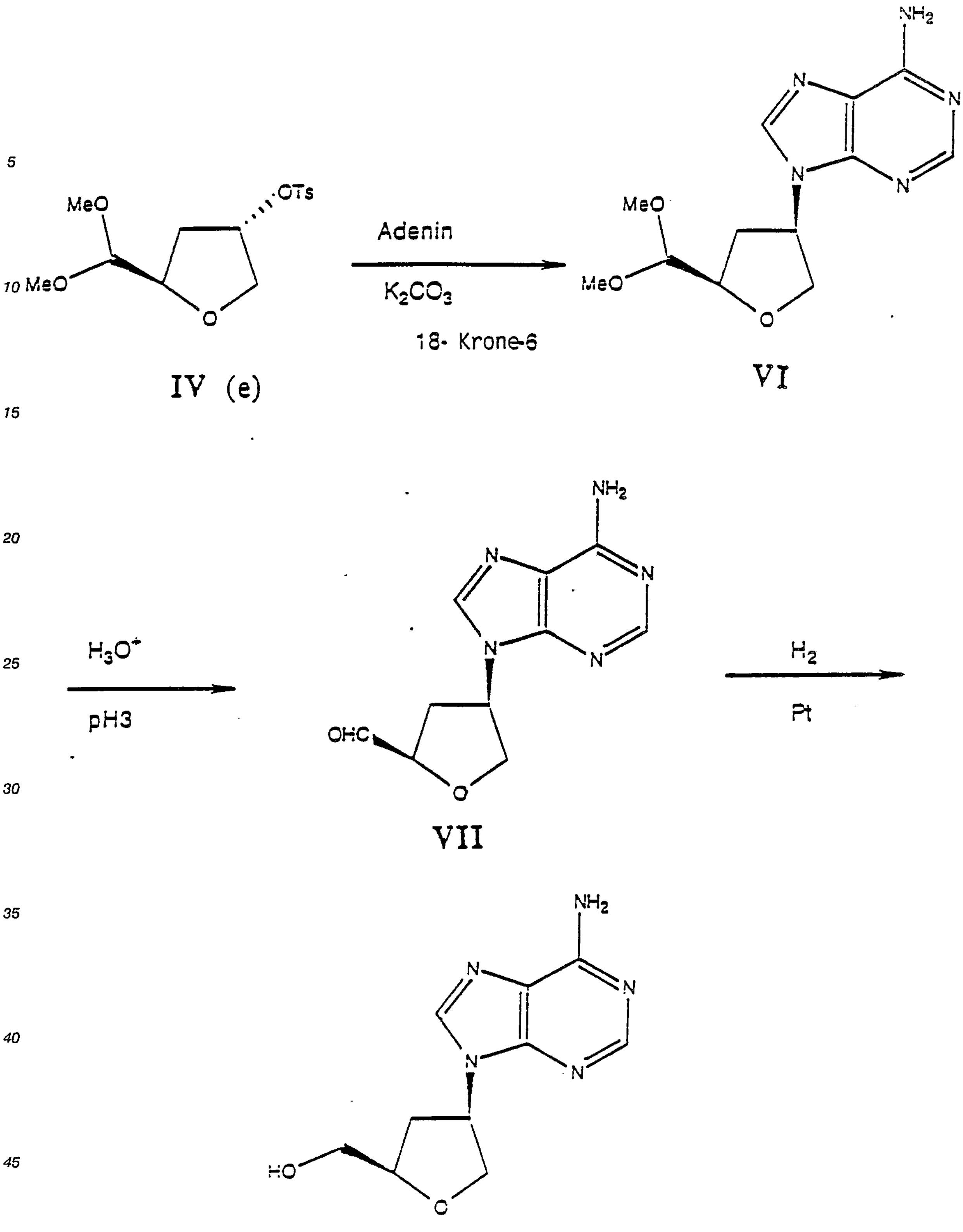


IV (e)

VI

VII

I a

Im obigen Reaktionsschema werden Adenin, Kaliumcarbonat und 18-Krone-6 mit dem Dimethylacetalisozucker zum Iso-ddA Acetal VI umgesetzt. Das Dimethylacetal wird dann mittels Oxalsäurelösung zum Aldehyd VII hydrolysiert. Nach Neutralisation wird der instabile Aldehyd sogleich katalytisch zum Iso-ddA Ia, umgesetzt. In diesem Reaktionsschema sind die Verbindungen IV(e), VI und VII neue Zwischenprodukte.

Die Verbindung Ib kann ebenfalls nach den oben angegebenen allgemeinen Methoden hergestellt werden. Die Verbindung IV(c) wird zunächst mit 2-Amino-6-chlorpurine, einem üblichen Phasentransferkatalysator wie 18-Krone-6 und Kaliumcarbonat zum neuen 2-Amino-6-chlorpurinisodideoxynucleosid VIII umgesetzt.

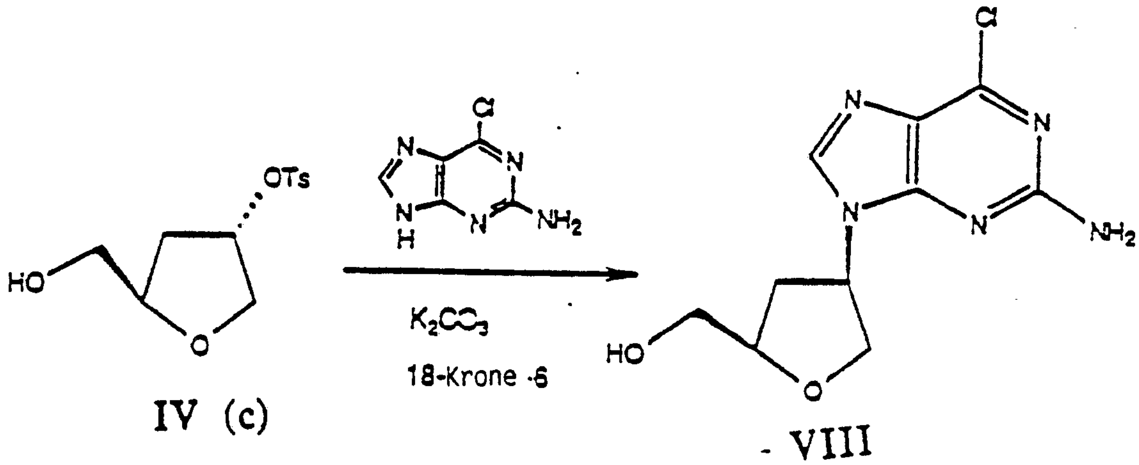


IV (c)

- VIII

Diese Verbindung wird dann in die Verbindung Ib entweder unter sauren oder unter basischen Bedingungen, beispielsweise durch Erhitzen in Gegenwart von HCl übergeführt

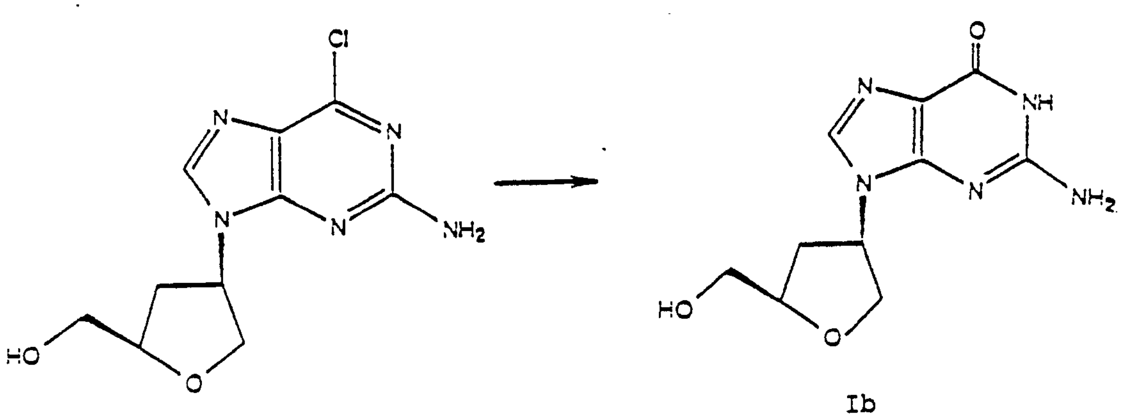

VIII

Ib

In diesem Schema ist die Verbindung VIII ein neues Zwischenprodukt.

Die isomeren Dideoxynukleoside der vorliegenden Erfindung können auch nach der klassischeren Methode hergestellt werden, bei der die Nukleobase aus einem Amin aufgebaut wird.

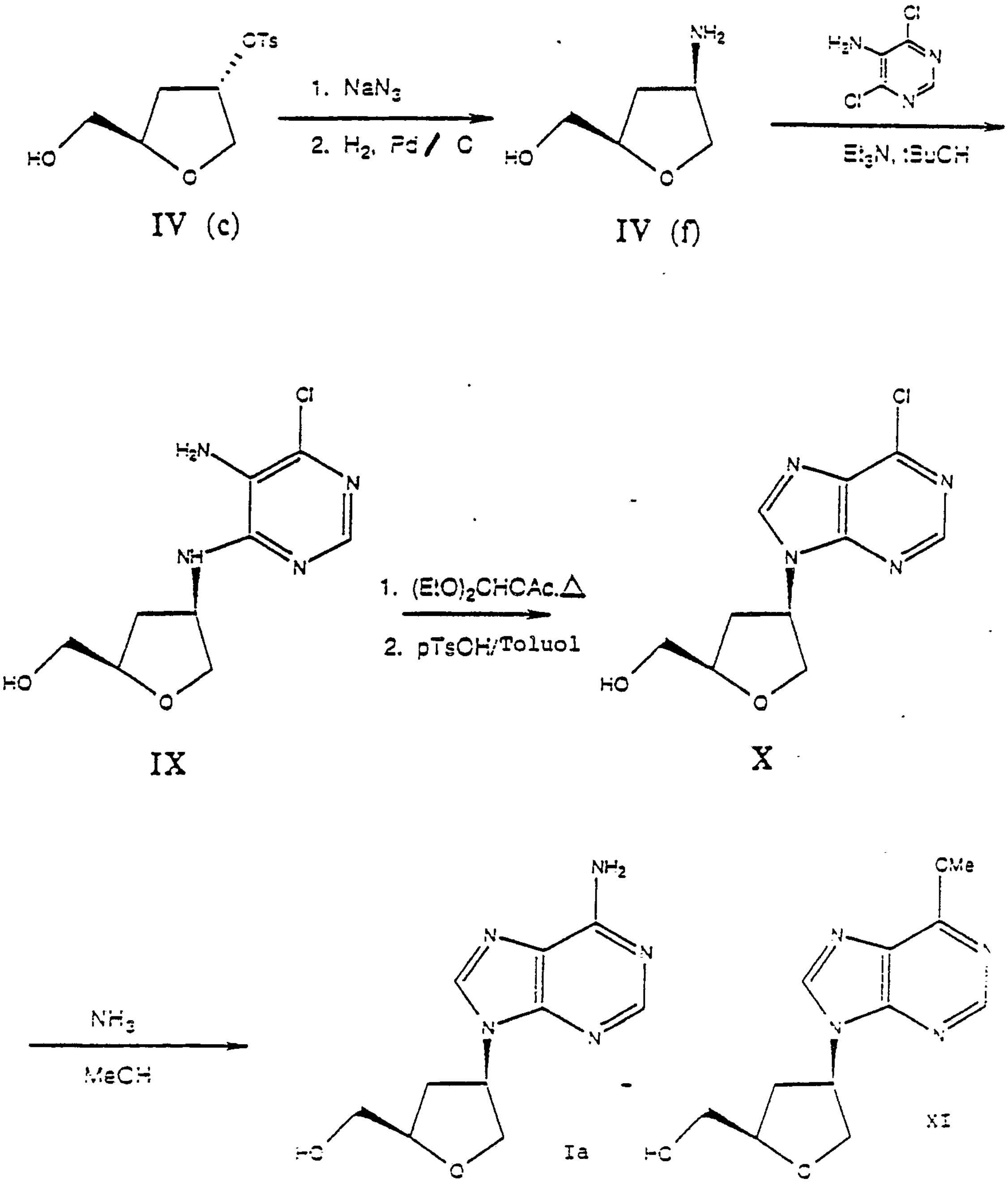


IV (c) IV (f) IX X Ia XI

In dem obigen Schema wird die Verbindung IV(c) mit Natriumazid umgesetzt. Die erhaltene Verbindung wird mit Palladium auf Kohle zum entsprechenden Amin IV(f) hydriert. Die Umsetzung dieses Amins mit 5-Amino-4,6-dichlorpyrimidin in Gegenwart einer Base liefert das Pyrimidinderivat IX, das wiederum in das Purinaddukt X durch Kondensation mit Dimethoxymethylacetat umgewandelt wird. Darauf folgt eine Säure-induzierte Eliminierung zum 6-Chlorpurinisodideoxynucleosid X. Anschliessende Behandlung mit methanolischer Ammoniaklösung liefert Iso-ddA Ib zusammen mit XI. In diesem Reaktionsschema sind die Verbindungen IX, X und XI neue Zwischenprodukte.

IsoddG kann ebenfalls aus der Verbindung IV(f) durch schrittweisen Aufbau der Nukleobase wie unten dargestellt, synthetisiert werden. Umsetzung von IV(f) mit 2-Amino-6-chlor-4(3H)-pyrimidinon in Gegenwart von Triäthylamin liefert das Pyrimidinon XII. Katalytische Reduktion von XII in Gegenwart von Platinoxid liefert das Aminopyrimidinon XIII. Behandlung von XIII mit Triäthylorthoformat und Säure liefert IsoddG Ib. Die Verbindungen XII und XIII sind neue Zwischenprodukte.

Die Derivate der Verbindung I, die an der Amino und der Hydroxygruppe substituiert sind, können in einfacher Weise aus der Verbindung der Formel I durch Umsetzung mit Anhydriden der allgemeinen Formel $(ArCO)_2O$, $(ArCH_2CO)_2O$ und $(RCO)_2O$ erhalten werden, wobei$(ArCO)_2O$ aromatische Säureanhydri-

de wie Benzoesäureanhydrid und $(ArCH_2CO)_2O$ araliphatische Säureanhydride wie Phenylessigsäureanhydrid darstellen und $(RCO)_2O$ aliphatische Säureanhydride darstellen. Das so erhaltene Produkt ist ein Ester-Amidderivat der Verbindung der Formel I. Selektive partielle Hydrolyse dieser Derivate mit Triäthylamin oder unter alkalischen Bedingungen liefert die gewünschte partiell substituierte Verbindung I.

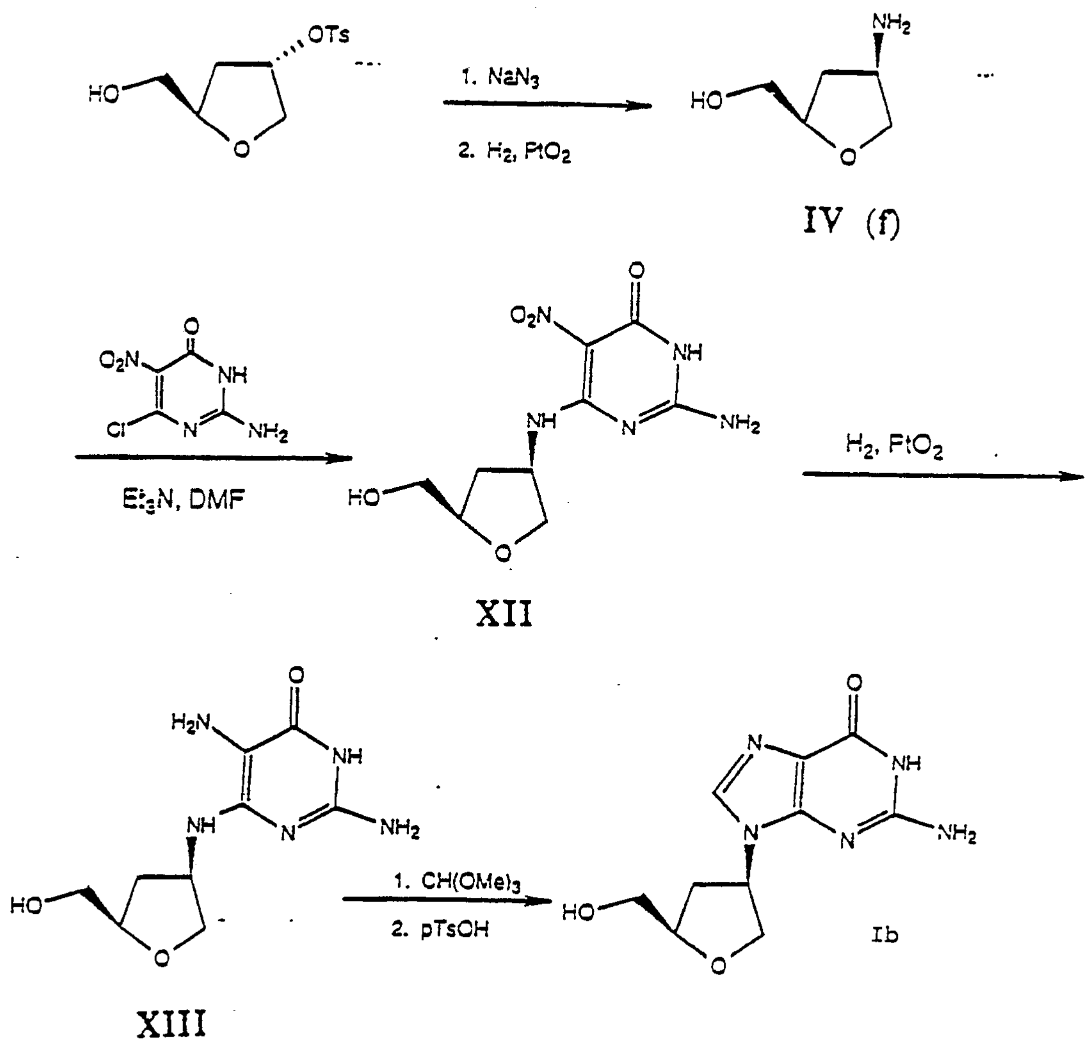


Die Verbindungen der Formel I können zur Behandlung von Retrovirusinfektionen, insbesondere HIV verwendet werden, wobei dem infizierten Organismus eine zur Inaktivierung des Virus hinreichende Menge der Verbindung oder eines ihrer pharmazeutisch anwendbaren Salze verabreicht wird.

Andere Dideoxynukleoside wie 2,3-Dideoxyadenosin und 2,3-Dideoxyinosin zeigen ebenfalls antiretrovirale Aktivität durch Hemmung der reversen Transkriptase. Der therapeutische Nutzen dieser Verbindungen ist durch deren raschen Abbau der Zucker-Basenbindung begrenzt. Die vorliegende Erfindung betrifft 2,3-Dideoxynucleosid-Isomere die eine stabilere Nukleosidbindung aufweisen.

Die Verbindungen der Formel I können in jeder geeigneten Verabreichungsweise angewandt werden, einschliesslich der oralen, der topischen und der parenteralen Verabreichung wie der subcutanen, intravenösen, intramuskulären oder intradermalen Verabreichung.

Obschon die Verbindung der Formel I allein verabreicht werden kann, ist es vorzuziehen, sie in Form pharmazeutischer Präparate anzuwenden. Die erfindungsgemässen Präparate beinhalten die Verbindung zusammen mit einem oder mehreren anwendbaren Trägern und gewünschtenfalls anderen therapeutischen Mitteln. Die pharmazeutischen Träger müssen anwendbar sein in dem Sinne, dass sie mit anderen Inhaltsstoffen der Präparate verträglich sind und dem Patienten nicht schaden.

Die erfindungsgemässen Präparate für orale Verabreichung können in Einheiten wie Kapseln, Beuteln oder Tabletten vorliegen, die jeweils eine bestimmte Menge der Verbindung I enthalten oder als Pulver oder Granulate, als Lösung oder Suspension in einer wässrigen oder nicht wässrigen Flüssigkeit oder als Oel-in-Wasser-Emulsion oder Wasser-in-Oel-Emulsion. Die Verbindungen der Formel I können auch als Bolus, Latwerge oder Paste vorliegen.

Tabletten können durch trockenes oder feuchtes Verpressen hergestellt werden und können z.B. durch Zusatz von Hydroxypropylmethylcellulose in variierenden Mengen ein kontrolliertes Freisetzungsprofil erhalten.

Geeignete Präparate zur parenteralen Verabreichung sind wässrige und nicht wässrige isotonische, sterile Injektionslösungen, die Antioxidantien, Puffer, Bakteriostatika und Mittel zur Erreichung der Isotonie enthalten können. Die Präparate können auch in lyophilisierter (gefriergetrockneter) Form vorliegen, aus der sie unmittelbar vor Gebrauch in sterile Injektionslösungen übergeführt werden können.

Die Erfindung wird durch die nachstehenden Beispiele weiter erläutert.

## Beispiel 1

Zu einer Lösung von 40 g 1,2-0-(1-Methyläthyliden)-α-D-xylofuranose in 125 ml Methylenchlorid werden 40 ml trockenes Pyridin gegeben. Die Lösung wird auf -5° C gekühlt und unter Stickstoff im Verlauf von 30 Minuten mit einer Lösung von 44,2 g Tosylchlorid in 100 ml Methylenchlorid versetzt. Nach Rühren über Nacht, wobei das Reaktionsgemisch auf Raumtemperatur aufgewärmt wurde, wurden 500 ml Methylenchlorid zugesetzt und das Reaktionsgemisch mit 1 x 600 ml Wasser, 2 x 400 ml Wasser und schliesslich mit 1 x 400 ml gesättigter Natriumchloridlösung gewaschen. Die organische Phase wurde getrocknet, filtriert und unter vermindertem Druck eingedampft. Nach weiterem Trocknen über Nacht bei 23° C/7 Pa zur Entfernung von restlichem Pyridin wurden 71 g Rohprodukt als Feststoff erhalten. Diese Substanz wurde in 100 ml Methylenchlorid gelöst und mit Hexan bis zur Trübung versetzt. Das Gemisch wurde über Nacht bei 0° C aufbewahrt. Der Feststoff wurde abfiltriert, mit Hexan gewaschen und an der Luft getrocknet wobei 52,4 g 1,2-0-(1-Methyläthyliden)-5-0-p-(tolylsulfonyl) -α-D-xylofuranose als weisser Feststoff erhalten wurden. Eine zweite Fraktion von 5,7 g wurde durch Eindampfen der Mutterlaugen erhalten (Ausbeute 80%).

Eine Lösung von 58,1 g 1,2-0-(1-Methyläthyliden)-5-0-p-(tolylsulfonyl) -α-D-xylofuranose und 1 l 1,2-Dichloräthan wurde mit 361 g Thiocarbonyldiimidazol versetzt. Die gelbe Lösung wurde 2 Stunden auf Rückflusstemperatur erhitzt, mit weiteren 4,2 g Thiocarbonyldiimidazol versetzt und eine weitere Stunde erhitzt. Da Reaktionsgemisch wurde gekühlt und das Lösungsmittel abgedampft. Der orange Rückstand wurde zwischen Methylenchlorid und 1,4 l Wasser verteilt. Die organische Phase wurde abgetrennt mit 700 ml Wasser und dann mit 500 ml gesättigter Natriumchloridlösung gewaschen, getrocknet, filtriert und eingedampft. Man erhielt 91 g 1,2-0-(1-Methyläthyliden)-α-D-xylofuranose-3-0-(1H-imidazol-1-carbothiosäure)-4-methylbenzolsulfonsäureester (Verbindung III) als orangen Feststoff.

## Beispiel 2

600 ml trockenes Toluol wurden mit Argon begast, auf Rückflusstemperatur erwärmt und mit 27,1 ml Tri-n-Butylzinnhydrid und 50 mg AIBN versetzt. Diese Lösung wurde im Verlauf einer Stunde mit einer Lösung von 30,3 g der in Beispiel 1 erhaltenen Verbindung in 600 ml trockenem Toluol gegeben. Nach weiteren 2 Stunden Erhitzen wurden Anteile einer Lösung von 9 ml Tri-n-Butylzinnhydrid und 50 mg AIBN unter weiterem Erhitzen zugesetzt bis die Dünnschichtchromatographie kein anwesendes Ausgangsmaterial mehr zeigte (etwa 1 Stunde später). Nach Abkühlen wurde das Toluol abgedampft, der Rückstand zwischen 1 l Acetonitril und 800 ml Hexan verteilt, die Acetonitrilphase eingedampft und die erhaltenen 27,3 g Rückstand in einem minimalen Volumen 30% Aethylacetat/70% Hexan gelöst. Das Gemisch wurde über Silicagel filtriert und mit 30% Aethylacetat/70% Hexan eluiert. Nach Abdampfen des Elutionsmittels wurde der Rückstand in einem geringen Volumen Aether gelöst und mit Petroläther versetzt, bis die Lösung sich trübte. Nach Kühlen im Trockeneisbad und Aufbewahren bei 0° C über Nacht wurden 9,3 g 3-Deoxy-1,2-0-(1-methyläthyliden)-α-D-erythro-pentofuranose-4-methylbenzolsulfonat als weisser Feststoff erhalten. Weitere 4 g Produkt wurden aus der Mutterlauge erhalten (Ausbeute 61%).

Eine Lösung von 12 g 3-Deoxy-1,2-0(1-methyläthyliden)-α-D-erythro-pentofuranose-4-methylbenzolsulfonat in 60 ml trockenem Methylenchlorid wurde unter Stickstoffatmosphäre auf -78° C gekühlt. Die Lösung wurde langsam mit 4 ml 1,3-Propandithiol und dann mit 5 ml Bortrifluoridätherat versetzt. Das Gemisch wurde 40 Stunden bei -78° C gerührt, sodann mit 200 ml Methylenchlorid versetzt, mit 2 x 50 ml gesättigter Natriumchloridlösung gewaschen, getrocknet, filtriert und eingedampft. Das leicht gefärbte Oel wurde durch

Säulenchromatographie mit 50% Aethylacetat/Hexan als Elutionsmittel gereinigt und lieferte 7 g (2S,4R)-4-(1,3-Dithian-2-yl)-1,2,4-butantriol-1-O-(4-methylbenzolsulfonat), (Verbindung V) als farbloses Oel (Ausbeute 50% oder 90% bezogen auf rückgewonnenes Ausgangsmaterial). Weiterhin wurden 5,3 g Ausgangsmaterial zurückgewonnen.

## Beispiel 3

Eine Lösung von 7 g der Verbindung V in 20 ml Methylenchlorid wurde mit 4 ml Pyridin versetzt, über Nacht gerührt, danach mit 50 ml Methylenchlorid versetzt und mit 20 ml gesättigter Natriumchloridlösung extrahiert. Die organische Phase wurde getrocknet, filtriert und eingedampft. Der Rückstand (Verbindung IV-(a)) wurde in 10 ml Methylenchlorid gelöst und mit 5,5 g Tosylchlorid in 3 ml Pyridin bei Raumtemperatur über Nacht gerührt. Nach Verdünnen mit 100 ml Methylenchlorid gefolgt von Waschen mit 25 ml Natriumchloridlösung, Trocknen und Filtrieren und Eindampfen und Chromatographie (30% Aethylacetat/70% Hexan) wurden 4,2 g (2R)-trans-Tetrahydro-5-(1,3-dithian-2-yl)-3-furanol -4-methylbenzolsulfonat (Verbindung IV(b)) als farbloses Oel erhalten: MS 360($M^+$)

## Beispiel 4

Ein Gemisch von 1 g (2R)-trans-Tetrahydro-5-(1,3-dithian-2-yl)-3-furanol-4-methylbenzolsulfonat (1,66 g) Quecksilber-(II)-Chlorid und 1,32 g Quecksilber-(II)-Oxid in 25 ml 80% Acetonitril/Wasser wurde 5 Stunden auf 80° C erwärmt. Die Lösung wurde auf Raumtemperatur gekühlt, filtriert und teilweise vom Acetonitril befreit. Der Rückstand wurde mit 50% Methanol/Wasser (10 ml) verdünnt, auf 0° C gekühlt und dann mit 100 mg Natriumborhydrid versetzt. Nach 15 Minuten Rühren bei 0° C wurde die Lösung konzentriert, der Rückstand zwischen Methylenchlorid und Kochsalz lösung verteilt, die organische Phase abgetrennt, getrocknet, filtriert und eingedampft. Das erhaltene Oel wurde durch Chromatographie mit 60% Aethylacetat/40% Hexan als Elutionsmittel gereinigt und lieferte 530 mg (2R-trans)-Tetrahydro-4-[[-(4methylphenyl) sulfonyl]oxy]-2-furanmethanol (Verbindung IV(c)) als Oel das beim Kühlen sich verfestigte: MS 241($M^+$-$CH_2OH$).

## Beispiel 5

Eine Lösung von 40 g 3-Deoxy-1,2-0-(1-methyläthyliden)-α-D-erythro-pentofuranose-4-methylbenzolsulfonat in 800 ml 1% (Volumen) Essigsäure in Methanol wurde 3 Tage auf 70° C erwärmt. Nach Kühlen auf Raumtemperatur wurde das pH der Lösung durch Zusatz von Natriummethoxidlösung auf 6,0 gestellt. Das Gemisch wurde dann unter vermindertem Druck zur Trockene eingedampft, wobei (3S-trans)-Tetrahydro-5-dimethoxymethyl-3-furanol (Verbindung IV(d)) als schaumiger Feststoff erhalten wurden. Diese Substanz wurde mit 160 ml trockenem Pyridin und 50 g Tosylchlorid versetzt und das Gemisch wurde 15 Stunden bei 0° C gerührt. Das Gemisch wurde dann in 400 ml Eiswasser gegossen, mit 2 x 400 ml Methylenchlorid extrahiert und nacheinander mit 1N Salzsäure, Natriumhydrogencarbonat und Wasser gewaschen. Die organische Lösung wurde dann zur Trockene eingedampft und lieferte 35,7 g (3S-trans)-Tetrahydro-5-dimethoxymethyl-3-furanol-4-methylbenzolsulfonat (Verbindung IV(e)) als bewegliche Flüssigkeit (93% Ausbeute).

IV(d): NMR (200 $MH_z$, $CDCl_3$) δ 2.00 (m, 2H), 3.45 (s, 6H), 3.78, 3.96 (AB of ABX, 2H, $J_{AB}$ = $9H_z$, $J_{AX}$ = $2H_z$, $J_{BX}$ = $4H_z$), 4.29 (m, 2H), 4.54 (m, 1H).

## Beispiel 6

Ein Gemisch von 10,0 g Verbindung IV(e), 50 ml Trifluoressigsäure und 80 ml Dioxan wurden 5 Stunden auf 80° C erwärmt. Nach Abkühlen auf Raumtemperatur wurde die Lösung durch Zusatz von 1N Natronlauge auf pH 7 gestellt, mit 1,2 g Natriumborhydrid versetzt und 30 Minuten bei Raumtemperatur

gerührt. Das Lösungsmittel wurde teilweise entfernt, und der Rückstand mit 3 x 100 ml Aethylacetat extrahiert. Die organische Phase wurde getrocknet, filtriert, eingedampft und mit 60% Aethylacetat/40% Hexan als Elutionsmittel chromatographiert. Man erhielt 3,6 g nicht umgesetztes Ausgangsmaterial und 2,7 g (2R-trans)-Tetrahydro-4-[[(4-methylphenyl)sulfonyl]oxy]-2-furanmethanol, (Verbindung IV(c)).

## Beispiel 7

50 mg Adenin, 100 mg Kaliumcarbonat, 97 mg 18-Krone-6 und 10 ml Dimethylformamid wurden 30 Minuten unter Argon bei Raumtemperatur gerührt. Zu dieser Lösung wurde tropfenweise eine Lösung von 100 mg (2R-trans)-Tetrahydro-4-[[(4-methylphenyl)sulfonyl]oxy]-2-furanmethanol, (Verbindung IV(c)) in 2 ml Dimethylformamid unter Rühren gegeben. Das erhaltene Gemisch wurde auf 80 °C erwärmt und bei dieser Temperatur über Nacht gerührt. Nach Entfernung des Lösungsmittels unter vermindertem Druck wurde der feste Rückstand durch Chromatographie mit 10% Methanol/90% Methylenchlorid als Elutionsmittel gereinigt. Das isolierte Material wurde durch Reversphasen HPLC mit einer Gradientelution mit Acetonitril/Wasser weiter gereinigt, wobei 25 mg (2R-cis)-4-(6-Amino-9H-purin-9-yl)tetrahydro-2-furanmethanol (Verbindung Ia) erhalten wurden. Ein Analysenpräparat wurde aus Methanol kristallisiert (Schmelzpunkt 183-185 °C $[\alpha]_D$ + 47,91 (c 0,67, Methanol).
UV λ max (MeOH) 260nm (ε 13990); UV λ max (pH 1) 210 nm (ε 19700), 259 nm (ε 13600).

## Beispiel 8

2,14 g Adenin, 4,37 g Kaliumcarbonat, 417 mg 18-Krone-6 und 400 ml Dimethylformamid wurden 30 Minuten unter Argon bei Raumtemperatur gerührt. Zu diesem gerührten Gemisch wurde tropfenweise eine Lösung von 5 g (3S-trans)-Tetrahydro-5-(dimethoxymethyl)-3-furanol-4-methylbenzolsulfonat, (Verbindung IV(e)) in 100 ml Dimethylformamid gegeben. Das erhaltene Gemisch wurde auf 80 °C erwärmt und bei dieser Temperatur über Nacht gerührt. Nach Filtrieren und Abdampfen des Lösungsmittels wurde ein fester Rückstand erhalten. Dieses Material wurde über Silicagel mit 7% Methanol/93% Methylenchlorid als Elutionsmittel gegeben. Das gewünschte Produkt enthaltenden Fraktionen wurden gesammelt und eingedampft und lieferten 3,0 g (3R-cis)-9-[Tetrahydro-5-(dimethyoxymethyl)-3-furanyl]-9H-purin-6-amin (Verbindung VI) Schmelzpunkt 144-146 °C.
UV λ max (MeOH) 214nm (ε 18890), 258 nm (ε 14100); UV λ max (pH1) 208 nm (ε 20000) 258 nm (ε 13780); UV λ max (pH 13) 259 nm (ε 14120).

## Beispiel 9

Eine Lösung von 150 mg Verbindung IV(c) in 3 ml getrocknetem Dimethylformamid wurde auf -5 °C gekühlt und mit 90 mg Natriumazid versetzt. Die Lösung wurde auf Raumtemperatur aufwärmen gelassen, dann 5 Stunden auf 70 °C erwärmt. Nach Entfernung des Dimethylformamids wurde der Rückstand zwischen 50 ml Aethylacetat und 10 ml Wasser verteilt. Die organische Phase wurde mit gesättigter Natriumbicarbonatlösung und gesättigter Kochsalzlösung gewaschen, die wässrigen Phasen wurden vereinigt, mit Aethylacetat zurückgewaschen, alle organischen Fraktionen wurden gesammelt, über Magnesiumsulfat getrocknet, filtriert und eingedampft. Das Material wurde mit 5 ml vorgetrocknetem Methanol und 15 mg 10% Palladium/Kohle 3 Stunden bei Raumtemperatur hydriert. Der Katalysator wurde abfiltriert und das Lösungsmittel abgedampft. Man erhielt 54 mg (2R-cis)-4-Aminotetrahydro-2-furanmethanol als farbloses Oel: MS 99($M^+$-$H_2O$).

## Beispiel 10

Ein Gemisch von 184 mg (2R-cis)-4-Aminotetrahydro-2-furanmethanol, 770 mg 5-Amino-4,6-dichlorpyrimidin, 0,44 ml Triäthylamin und 16 ml t-Butylalkohol wurde unter Argon 72 Stunden zum Rückfluss

erhitzt, worauf die flüchtigen Stoffe abgedampft wurden. Der Rückstand wurde in 100 ml Aethylacetat gelöst, die Lösung mit gesättigter Natriumhydrogencarbonatlösung und gesättigter Kochsalzlösung gewaschen, getrocknet, filtriert und eingedampft. Das erhaltene Produkt wurde durch Säulenchromatographie (50% Aethylacetat/50% Hexan) gereinigt und lieferte 220 mg (2R-cis)-4-[(5-Amino-6-chlor-4-pyrimidinyl)-amino] tetrahydro-2-furanmethanol (Verbindung IX).
UV λ max (MeOH) 206 nm, 265 nm, 295 nm; UV λ max (pH 1) 204 nm, 304 nm.

## Beispiel 11

Eine Lösung von 220 mg der Verbindung IX und 5 ml Diäthoxymethylacetat wurde 120 Stunden auf 100 °C erwärmt. Die flüchtigen Stoffe wurden dann abgedampft und der Rückstand mit 5 ml Toluol und 5 mg p-Toluolsulfonsäure versetzt. Nach Rühren bei Raumtemperatur während einer Stunde und Entfernung des Toluols wurde der Rückstand durch Säulenchromatographie (10% Methanol/90% Methylenchlorid) gereinigt und lieferte 109 mg (2R-cis)-4-(6-Chlor-9H-purin-9-yl)tetrahydro-2-furanmethanol (Verbindung X) als weissen Feststoff.
UV λ max (MeOH) 264 nm; UV λ max (pH1) 265 nm;

## Beispiel 12

Eine Lösung von 73 mg (2R-cis)-4-(6-Chlor-9H-purin-9-yl) tetrahydro-2-furanmethanol in 15 ml 16% $NH_3$/Methanol wurde in einem Edelstahl-Autoklaven 96 Stunden reagieren gelassen. Nach Abdampfen der Lösungsmittel und Säulenchromatographie (10% Methanol/90% Methylenchlorid) wurden 20 mg (2R-cis)-4-(6-Amino-9H-purin-9-yl)tetrahydro-2-furanmethanol (Verbindung Ia) und 38 mg (2R-cis)-Tetrahydro-4-(6-methoxy-9H-purin-9-yl)-2-furanmethanol (Verbindung XI) als weisser Feststoff erhalten.
XI: UV λ max (MeOH) 249 nm; UV λ max (pH1) 250 nm.

## Beispiel 13

Ein Gemisch von 63 mg 2-Amino-6-chlor-purin, 100 mg Kaliumcarbonat, 97 mg 18-Krone-6 und 10 ml Dimethylformamid wurde unter Argon 60 Minuten bei Raumtemperatur gerührt. Danach wurde eine Lösung von 100 mg (2R-trans)-Tetrahydro-4-[(4-methylphenyl)sulfonyl]oxy-2-furan-methanol in 2 ml Dimethylformamid tropfenweise unter Rühren zugesetzt. Das erhaltene Gemisch wurde auf 80 °C erwärmt und bei dieser Temperatur über Nacht gerührt. Nach Entfernung des Lösungsmittels unter vermindertem Druck wurde der feste Rückstand durch Säulenchromatographie mit 5% Methanol/95% Methylenchlorid als Elutionsmittel gereinigt. Das isolierte Material wurde weiter gereinigt durch Reversphasen-HPLC mit einem Gradientelutionssystems von Acetonitril/Wasser und lieferte 30 mg (2R-cis)-4-(2-Amino-6-chlor-9H-purin-9-yl)tetrahydro-2-furanmethanol (Verbindung VIII) als weissen Feststoff: UV λmax (EtOH) 221 nm, 240 nm, 303 nm.

## Beispiel 14

Eine Lösung von 4,35 g (3R-cis)-9-[Tetrahydro-5-(dimethoxymethyl)-3-furanyl]-9H-purin-6-amin in 200 ml 0,1M Oxalsäurelösung wurde 18 Stunden zum Rückfluss erhitzt und lieferte (2R-cis)-4-(6-Amino-9H-purin-9-yl)tetrahydro-2-furancarbaldehyde (Verbindung VII). Nach Abkühlen auf Raumtemperatur wurde die die Verbindung VII enthaltende Lösung mit 1N Natronlauge neutralisiert. Das erhaltene Gemisch wurde mit 50 mg $PtO_2$ versetzt und unter Wasserstoffatmosphäre 48 Stunden gerührt. Nach Filtrieren, Eindampfen und Reinigung durch Säulenchromatographie (5% Methanol/95% Methylenchlorid) wurden 2,0 g (2R-cis)-4-(6-Amino-9H-purin-9-yl)tetrahydro-2-furanmethanol (Verbindung Ia) als weisser Feststoff erhalten. Gewünschtenfalls kann das Produkt durch Umkristallisation aus Methanol weiter gereinigt werden.

Beispiel 15

Eine Lösung von 170 mg (2R-cis)-4-(2-Amino-6-chlor-9H-purin-9-yl)-tetrahydro-2-furanmethanol in 25 ml 1N Salzsäure wurde 3 Stunden zum Rückfluss erhitzt. Das Reaktionsgemisch wurde mit 40% Kalilauge neutralisiert und dann zu einem weissen Feststoff eingedampft. Reinigung durch Reversphasen-HPLC mit Acetonitril/Wasser-Gradientelution lieferte 65 mg (3R-cis)-2-Amino-1,9-dihydro-9-[tetrahydro-5-(hydroxymethyl)-3-furanyl]-6H-purin-6-on (Verbindung Ib) als weissen Feststoff, Schmelzpunkt 229-330 °C; UV λ max (pH 0) 205 nm, 253 nm, 278 nm; UV λ max (pH 6) 252 nm, 270 nm; UV λ max (pH 13) 255 nm, 268 nm.

Beispiel 16

427 mg (2R-cis)-4-Aminotetrahydro-2- furanmethanol, 540 mg 2-Amino-5-nitro-6-chlor-4(3H)pyrimidinon, 3,5 ml Triäthylamin und 5 ml Dimethylformamid wurden 6 Stunden auf 60 °C erwärmt. Danach wurde auf Raumtemperatur abgekühlt, das Reaktionsgemisch in Gegenwart von Silicagel eingedampft, das Material auf eine Silicagelsäule gebracht und mit 5:1 Methylenchlorid/Methanol und danach mit 3:1 Methylenchlorid/Methanol eluiert, wobei 730 mg der Verbindung XII erhalten wurden. 200 mg der Verbindung XII wurden mit 75 mg $PtO_2$ und 50 ml einer 1:3 (Volumen)-Lösung von Ameisensäure und Methanol versetzt und 48 Stunden mit 3,75 bar hydriert. Nach Filtrieren und Eindampfen wurde die Verbindung XIII erhalten. Der Rückstand wurde mit 5 ml Dimethylformamid, 5 ml Triäthylorthoformat und 200 mg p-Toluolsulfonsäure versetzt und 4 Stunden auf 80 °C erwärmt. Die Lösung wurde dann eingedampft und mit 10 ml 0,5N Salzsäure 4 Stunden gerührt. Nach Neutralisation und Eindampfen des Gemisches und Reinigung wurde die Verbindung Ib erhalten.

Beispiel 17

Die Verbindungen Ia und Ib wurden auf ihre anti-HIV-Aktivität nach dem von Mitsuya, et al. in Proc. Nat'l Acad. Sci., USA, 83:1911 (1986) beschriebenen Test geprüft.

Die Zellen wurden entweder mit der Verbindung Ia, der Verbindung Ib, oder 3-Azido-2,3-dideoxythymidin (AZT) zusammengebracht und die überlebenden Zellen wurden nach 7 Tagen bestimmt.

Die Resultate sind in Figur I dargestellt. Die schwarzen Säulen repräsentieren die überlebenden, behandelten Zellen bei Anwesenheit von HIV (HTLV-III). Die hellen Säulen repräsentieren die überlebenden, behandelten Zellen in Abwesenheit des Virus.

Die Verbindungen Ia und Ib zeigen eine anti-HIV-Aktivität, die äquivalent zu der Aktivität bekannter antiretroviraler Therapeutika ist, die in vivo gegen HIV wirksam sind, insbesondere AZT

Beispiel 18

Prüfung der Verbindungen Ia und Ib auf anti-HIV-Aktivität im ELISA-Testsystem

Der ELISA (Enzyme Linked Immuno-absorbant Assay)-Test wurde zur Prüfung der anti-HIV-Aktivität der Verbindung Ia und Ib benutzt, wobei das Viruswachstum in $CD4^+$ T-Lymphocyten durch Nachweis des Antigens gemessen wurde, das in den Ueberstand der Gewebskultur infizierter Zellen abgegeben wurde.

Viruspräparate mit hohem Titer (HIV, HTLV III, RF-Stamm) wurden in H9-Zellen in RPMI 1640 (Flow Laboratories) das mit 10% fötalem Kälberserum, 100 IU/ml Penicillin und 100 µg/ml Streptomycin ergänzt war, angezüchtet. Die Zelltrümmer wurden durch niedertouriges Zentrifugieren entfernt und der Ueberstand bei -70 °C bis zum Gebrauch aufbewahrt. Die verwendete Wirtszelle war eine C8166 $CD4^+$ T-Zellinie, die besonders empfindlich gegenüber Infektionen mit HIV ist. Die Zellen wurden mit 10 $TCID_{50}$ HIV/HTLV III-(RF) 90 Minuten bei 37 °C inkubiert und dann dreimal mit PBSA gewaschen. Aliquote Teile (2 x $10^5$ Zellen)

wurden in 1,5 ml Nährmedium in 6 ml Röhrchen bei 37° C inkubiert. 72 Stunden nach der Infektion wurden die Ueberstände aus den Zellkulturen sofort auf HIV-Antigen geprüft.

Für den Antigennachweis wurde ein ELISA von Coulter (Luton, UK) verwendet. Die Teste wurden mit Ueberständen aus infizierten Zellkulturen nach Vorschrift des Herstellers ausgeführt. Bei jeder Testreihe wurden entsprechende positive und negative interne Kontrollen zusammen mit einer Kontrolle aus dem Ueberstand nicht infizierter Zellen eingeschlossen. Die Kulturplatten wurden mit einem entsprechenden spektrophotometrischen Detektorsystem ausgewertet.

Die Resultate des ELISA-Tests mit der Verbindung Ia und ddA (ein bekanntes anti-HIV-Mittel) ist nachstehend angegeben.

| $IC_{50}$ (µM) | Verbindung |
|---|---|
| 3-10 | Ia |
| 1-3 | ddA |

Die Verbindung Ia zeigte in diesem Testsystem eine dem bekannten anti-HIV-Mittel ddA vergleichbare antivirale Aktivität.

## Beispiel 19

### Tablettenformulierungen für die Verbindung Ia und Ib

| A. 0,05 mg-Tablette | |
|---|---|
| Inhaltsstoffe | mg/Tablette |
| 1. Verbindung Ia oder Verbindung Ib | 0,05 |
| 2. Laktose wasserfrei | 94,00 |
| 3. Eisenoxid schwarz | 0,15 |
| 4. Mikrokristalline Cellulose (Avicel PH-102) | 25,00 |
| 5. Croscarmellose Natrium, Type A (Ac-Di-Sol) | 5,00 |
| 6. Magnesiumstearat | 0,80 |
| TOTAL | 125,00 mg |

| B. 2 mg-Tablette | |
|---|---|
| Inhaltsstoffe | mg/Tablette |
| 1. Verbindung Ia oder Verbindung Ib | 2,00 |
| 2. Laktose wasserfrei | 147,00 |
| 3. Kosmetisches Eisenoxid gelb | 0,40 |
| 4. Mikrokristalline Cellulose (Avicel PH-102) | 40,00 |
| 5. Croscarmellose Natrium, Type A (Ac-Di-Sol) | 8,40 |
| 6. Magnesiumstearat | 2,20 |
| TOTAL | 200,00 mg |

## Beispiel 20

Injizierbare Formulierungen für die Verbindungen Ia und Ib

A. Steriles Pulver für Injektionen

| 1. Verbindung Ia oder Ib als lyophilisiertes Pulver, 10 mg/Ampulle | |
|---|---|
| Inhaltsstoffe | mg/Ampulle |
| 1. Verbindung Ia oder Verbindung Ib<br>2. Mannit<br>3. Salzsäure (1 Volumenprozent)<br>4. Wasser für Injektionszwecke | 10.00<br>50.00 |
| a. Die Salzsäurelösung wird zur Einstellung des pH der Stammlösung vor der Gefriertrocknung verwendet. | |

B. Pulver zur Herstellung von Lösungen

| 1. Verbindung Ia oder Verbindung Ib 0,5 mg/Pulver | |
|---|---|
| Inhaltsstoffe | mg |
| 1. Verbindung Ia oder Verbindung Ib<br>2. Laktose wasserfrei<br>TOTAL | 0,50<br>99,50<br>100,00 mg |

Figur I

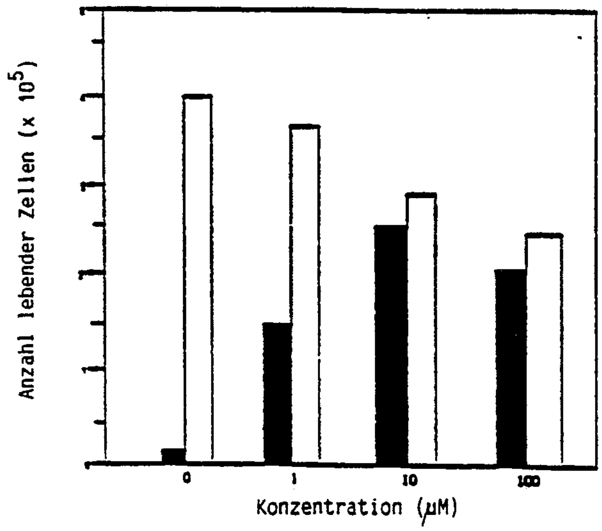


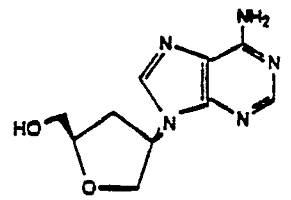

■ Virus-infizierte Zellen

□ nicht infizierte Zellen

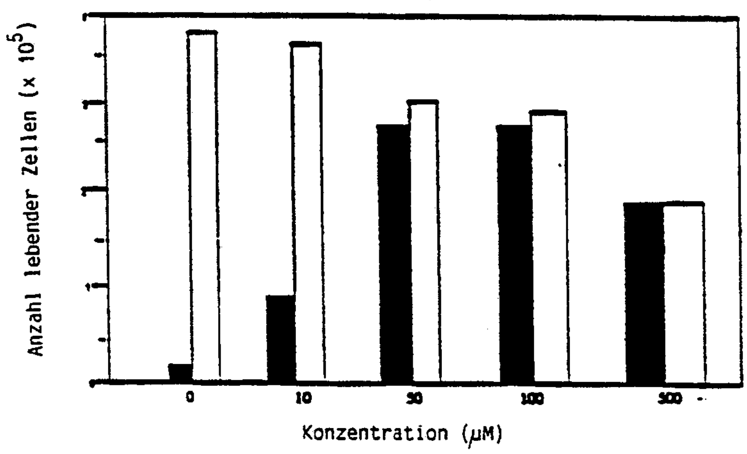


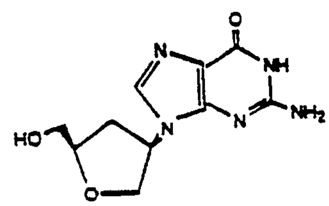

■ Virus-infizierte Zellen

□ nicht infizierte Zellen

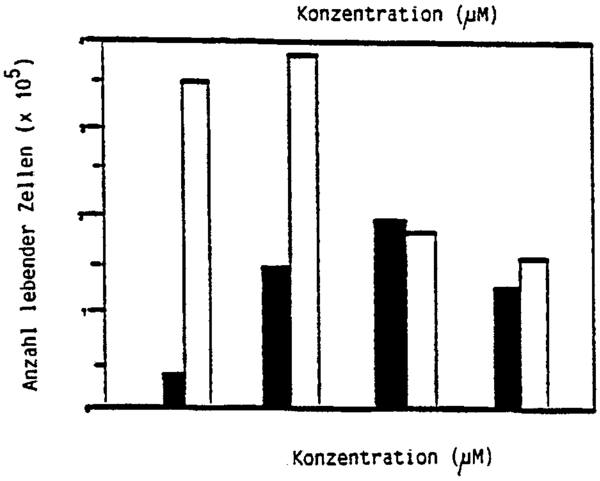


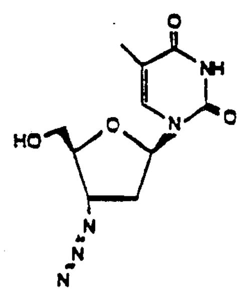

■ Virus-infizierte Zellen

□ nicht infizierte Zellen

**Ansprüche**

1. Verbindungen der Formel

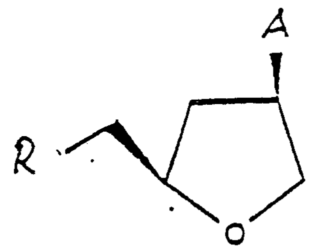

I

worin A 6-Amino-9H-purin-9-yl oder 2-Amino-6-hydroxy-9H-purin-9-yl darstellt, worin die Aminogruppe durch $NHC(O)R^2$ oder $N=CHR^3R^4$ ersetzt sein kann und R OH oder $OC(O)R^1$ ist, wobei $R^1$, $R^2$, $R^3$ und $R^4$ unabhängig voneinander geradkettiges oder verzweigtes $C_{1-20}$-Alkyl, $C_{1-12}$-Alkoxyalkyl, substituiertes oder unsubstituiertes Phenoxy-$C_{1-4}$-alkyl, Phenyl oder Phenyl das durch Halogen, $C_{1-4}$-Alkoxy, Nitro, Hydroxy, Carboxyl oder Alkylamino substituiert ist, bedeuten und pharmazeutisch anwendbare Salze davon.

2. Verbindungen nach Anspruch 1, wobei A unsubstituiertes 6-Amino-9H-purin-9-yl ist

3. (2R-cis)-4-(6-Amino-9H-purin-9-yl)-tetrahydro-2-furanmethanol.

4. Verbindungen nach Anspruch 1, wobei A unsubstituiertes 2-Amino-6-hydroxy-9H-purin-9-yl ist.

5. (3R-cis)-2-Amino-1,9-dehydro-9-[tetrahydro-5-(hydroxymethyl)-3-furanyl] 6H-purin-6-on.

6. Verbindungen der Formel

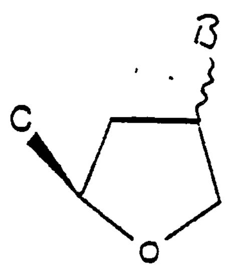

IV

wobei B $NH_2$, Hydroxy oder eine abgewandelte Form davon oder deren maskierte Vorläufer oder eine Abgangsgruppe bedeuten und C Hydroxymethyl oder einen maskierten Vorläufer davon bedeuten.

7. Die Verbindungen
(2R-trans)-Tetrahydro-5-(1,3-dithian-2-yl)-3-furanol;
(2R-trans)-Tetrahydro-5-(1,3-dithian-2-yl)-3-furanol-4-methylbenzolsulfonat;
(2R-trans)-Tetrahydro-4-[[(4-methylphenyl)sulfonyl]oxy]-2-furanmethanol;
(3S-trans)-Tetrahydro-5-dimethoxymethyl-3-furanol;
(3S-trans)-Tetrahydro-5-dimethoxymethyl-3-furanol-4-methylbenzolsulfonat; und
(2R-cis)-4-Aminotetrahyxdro-2-furanmethanol.

8. 1,2-0-(1-Methylethyliden)-α-D-xylofuranose 3-0-(1H-imidazol-1-carbothiosäure)-4-methylbenzolsulfonsäureester.

9. (2S,4R)-4-(1,3-Dithian-2-yl)-1,2,4-butantriol-1-0-(4-methylbenzolsulfonat).

10. (3R-cis)-9-[Tetrahydro-5-(dimethyoxymethyl)-3-furanyl]-9H-purin-6-amin.

11. (2R-cis)-4-(6-Amino-9H-purin-9-yl)tetrahydro-2-furancarbaldehyd.

12. (2R-cis)-4-(2-Amino-6-chlor-9H-purin-9-yl)tetrahydro-2-furanmethanol.

13. (2R-cis)-4-[(5-Amino-6-chlor-4-pyrimidinyl)amino]tetrahydro-2-furanmethanol.

14. (2R-cis)-4-(6-Chlor-9H-purin-9-yl)tetrahydro-2-furanmethanol.

15. (2R-cis)-Tetrahydro-4-(6-methoxy-9H-purin-9-yl)-2-furanmethanol.

16. (2R-cis)-2-Amino-5-nitro-6-[[tetrahydro-5-(hydroxymethyl)-3-furanyl]amino]-4(3H)pyrimidinon.

17. (2R-cis)-2,5-Diamino-6-[[tetrahydro-5-(hydroxymethyl)-3-furanyl]amino]-4(3H)-pyrimidinon.

18. Die Verbindungen der Ansprüche 1-5 zur Verwendung als Heilmittel.

19. Verfahren zur Herstellung der Verbindungen von Anspruch 1, dadurch gekennzeichnet, dass man a) eine Verbindung der Formel

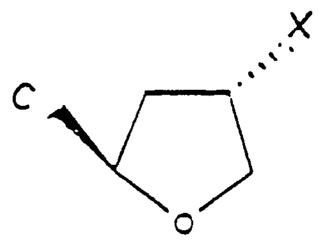

IV-1

worin X eine abspaltbare Gruppe und C Hydroxymethyl oder ein maskierter Vorläufer davon ist, mit Adenin oder Guanin umsetzt und erforderlichenfalls einen maskierten Vorläufer C in eine Hydroxymethylgruppe überführt oder

b) den Chlorsubstituenten in einer Verbindung der Formel

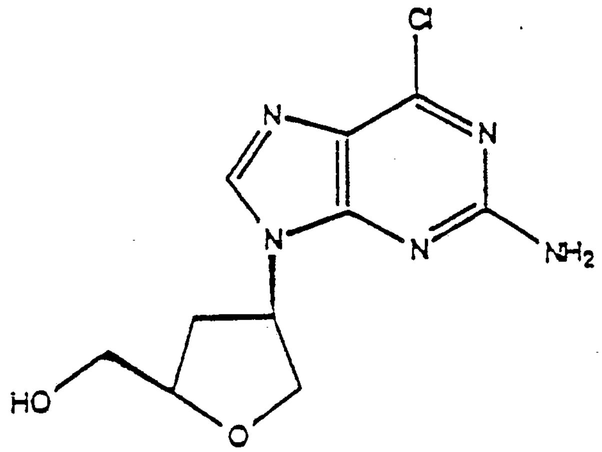

VIII

hydrolysiert oder

c) eine Verbindung der Formel

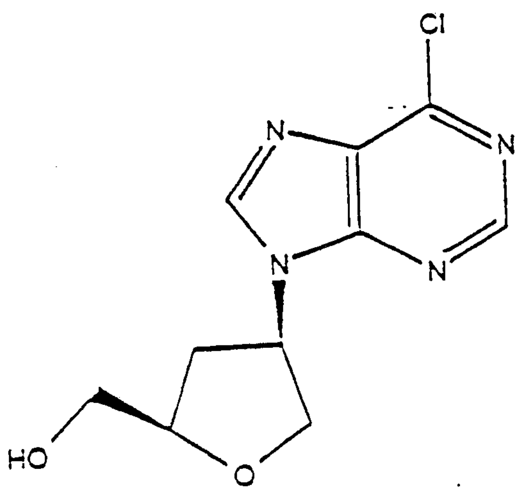

X

mit Ammoniak umsetzt oder

d) eine Verbindung der Formel

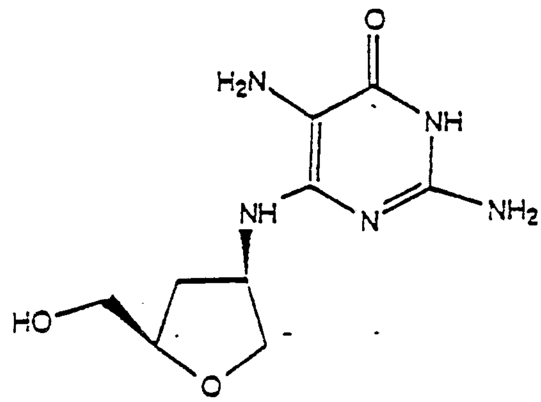

XIII

mit einem Orthoameisensäureester oder mit Diäthoxymethylacetat umsetzt
und gewünschtenfalls die erhaltene Verbindung der Formel I in ein pharmazeutisch anwendbares Salz oder ein N- oder O-Acyl-Derivat oder eine Schiff'sche Base umwandelt.

20. Pharmazeutische Präparate, enthaltend eine Verbindung nach Anspruch 1-5 und pharmazeutische Hilfs- oder Trägerstoffe.

21. Verwendung von Verbindungen nach Anspruch 1-5 zur Herstellung von Heilmitteln zur Behandlung retroviraler Infektionen, wie HIV-Infektionen.

Patentansprüche für folgende Vertragsstaaten: ES, GR

1. Verfahren zur Herstellung von Verbindungen der Formel

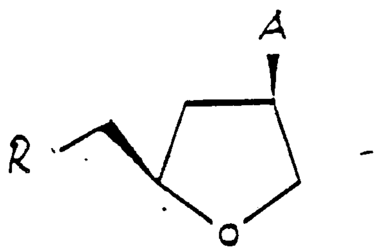

I

worin A 6-Amino-9H-purin-9-yl oder 2-Amino-6-hydroxy-9H-purin-9-yl darstellt, worin die Aminogruppe durch $NHC(O)R^2$ oder $N=CHR^3R^4$ ersetzt sein kann und R OH oder $OC(O)R^1$ ist, wobei $R^1$, $R^2$, $R^3$ und $R^4$ unabhängig voneinander geradkettiges oder verzweigtes $C_{1-20}$-Alkyl, $C_{1-12}$-Alkoxyalkyl, substituiertes oder unsubstituiertes Phenoxy-$C_{1-4}$-alkyl, Phenyl oder Phenyl das durch Halogen, $C_{1-4}$-Alkoxy, Nitro, Hydroxy, Carboxyl oder Alkylamino substituiert ist, bedeuten und pharmazeutisch anwendbare Salze davon, dadurch gekennzeichnet, dass man

a) eine Verbindung der Formel

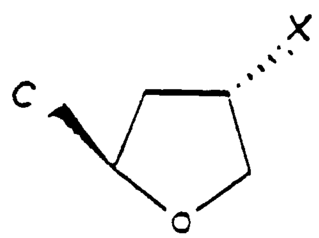

IV-1

worin X eine abspaltbare Gruppe und C Hydroxymethyl oder ein maskierter Vorläufer davon ist, mit Adenin oder Guanin umsetzt und erforderlichenfalls einen maskierten Vorläufer C in eine Hydroxymethylgruppe überführt oder

b) den Chlorsubstituenten in einer Verbindung der Formel

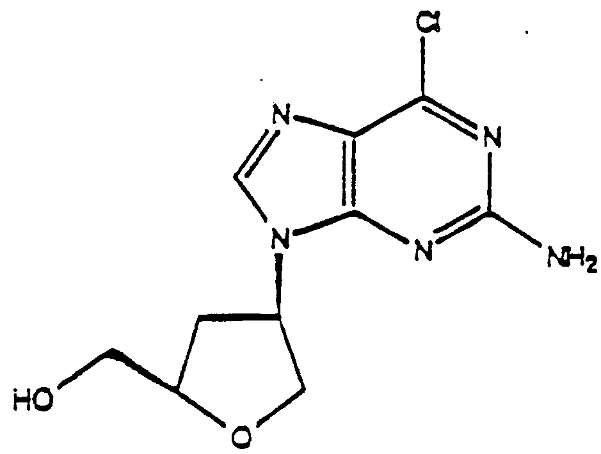

VIII

hydrolysiert oder
c) eine Verbindung der Formel

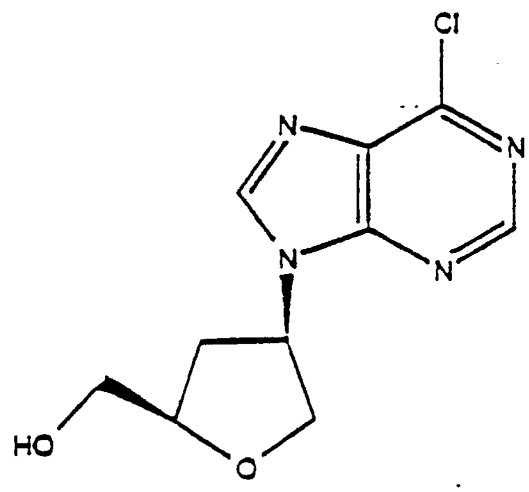

X

mit Ammoniak umsetzt oder
d) eine Verbindung der Formel

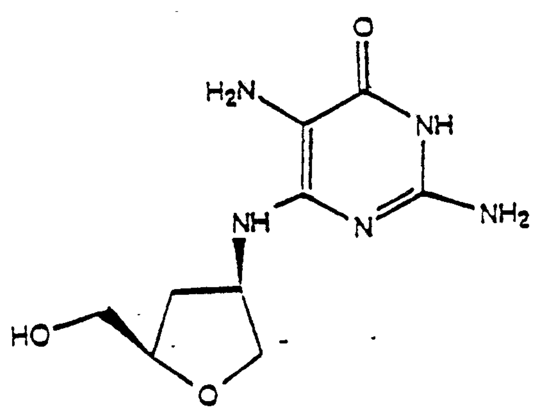

XIII

mit einem Orthoameisensäureester oder mit Diäthoxymethylacetat umsetzt
und gewünschtenfalls die erhaltene Verbindung der Formel I in ein pharmazeutisch anwendbares Salz oder ein N- oder O-Acyl-Derivat oder eine Schiff'sche Base umwandelt.

2. Verfahren nach Anspruch 1 zur Herstellung von Verbindungen der Formel I, worin A unsubstituiertes 6-Amino-9H-purin-7-yl ist.

3. Verfahren nach Anspruch 1 zur Herstellung von (2R-cis)-4-(6-Amino-9H-purin-9-yl)-tetrahydro-2-furanmethanol.

4. Verfahren nach Anpruch 1 zur Herstellung von Verbindungen der Formel I, worin A unsubstituiertes 2-Amino-6-hydroxy-9H-purin-9-yl ist.

5. Verfahren nach Anspruch 1 zur Herstellung von (3R-cis)-2-Amino-1,9-dihydro-9-[tetrahydro-5-(hydroxymethyl)-3-furanyl]-6H-purin-6-on.

6. Verfahren nach Anspruch 1, wobei (2R-trans)-Tetrahydro-4-[[(4-methylphenyl)sulfonyl]oxy]-2-furanmethanol mit Adenin in Gegenwart von 18-Krone-6 und Kaliumcarbonat in Dimethylformamid zu (2R-cis)-4-(6-Amino-9H-purin-9-yl)-tetrahydro-2-furanmethanol umgesetzt wird.

7. Verfahren nach Anspruch 1, wobei (2R-cis)-4-(6-Chlor-9H-purin-9-yl)-tetrahydro-2-furanmethanol mit Ammoniak in Methanol zu (2R-cis)-4-(6-Amino-9H-purin-9-yl)tetrahydro-2-furanmethanol umgesetzt wird.

8. Verfahren nach Anspruch 1, wobei (2R-cis)-4-(6-Amino-9H-purin-9-yl)tetrahydro-2-furancarbaldehxd in Gegenwart von $PtO_2$ zu (2R-cis)-4-(6-Amino-9H-purin-9-yl)tetrahydro-2-furanmethanol hydriert wird.

9. Verfahren nach Anspruch 1, wobei (2R-cis)-4-(2-Amino-6-chlor-9H-purin-9-yl)tetrahydro-2-furanmethanol mit Salzsäure erhitzt wird und (3R-cis)-2-Amino-1,9-dihydro-9-[tetrahydro-5-(hydroxymethyl)-3-furanyl]-6H-purin-6-on liefert.

10. Verfahren nach Anspruch 1, wobei (2R-cis)-2,5-Diamino-6-[[tetrahydro-5-(hydroxymethyl)-3-furanyl]-amino]-4(3H)-pyrimidinon mit Triäthylorthoameisenester und p-Toluolsulfonsäure zu (3R-cis)-2-Amino-1,9-dihydro-9-[tetrahydro-5-(hydroxymethyl)-3-furanyl]-6H-purin-6-on umgesetzt wird.

11. Verfahren zur Herstellung pharmazeutischer Präparate, dadurch gekennzeichnet, dass man eine Verbindung von Anspruch 1-5 mit einem pharmazeutischen Hilfs- oder Trägerstoff vermischt und in eine galenische Anwendungsform bringt.

12. Verwendung von Verbindungen nach Anspruch 1-5 zur Herstellung von Heilmitteln zur Behandlung retroviraler Infektionen, insbesondere HIV-Infektionen.

13. Verbindungen der Formel

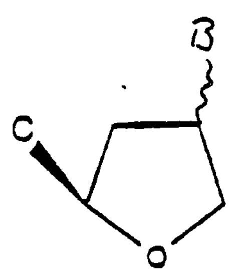

IV

wobei B $NH_2$, Hydroxy oder eine abgewandelte Form davon oder deren maskierte Vorläufer oder eine Abgangsgruppe bedeuten und C Hydroxymethyl oder einen maskierten Vorläufer davon, bedeuten.

14. Die Verbindungen

(2R-trans)-Tetrahydro-5-(1,3-dithian-2-yl)-3-furanol;
(2R-trans)-Tetrahydro-5-(1,3-dithian-2-yl)-3-furanol-4-methylbenzolsulfonat;
(2R-trans)-Tetrahydro-4-[[(4-methylphenyl)sulfonyl]oxy]-2-furanmethanol;
(3S-trans)-Tetrahydro-5-dimethoxymethyl-3-furanol;
(3S-trans)-Tetrahydro-5-dimethoxymethyl-3-furanol-4-methylbenzolsulfonat; und
(2R-cis)-4-Aminotetrahyxdro-2-furanmethanol.

15. 1,2-0-(1-Methylethyliden)-α-D-xylofuranose 3-0-(1H-imidazol-1-carbothiosäure)-4-methylbenzolsulfonsäureester.

16. (2S,4R)-4-(1,3-Dithian-2-yl)-1,2,4-butantriol-1-0-(4-methylbenzolsulfonat).

17. (3R-cis)-9-[Tetrahydro-5-(dimethyoxymethyl)-3-furanyl]-9H-purin-6-amin.

18. (2R-cis)-4-(6-Amino-9H-purin-9-yl)tetrahydro-2-furancarbaldehyd.

19. (2R-cis)-4-(2-Amino-6-chlor-9H-purin-9-yl)tetrahydro-2-furanmethanol.

20. (2R-cis)-4-[(5-Amino-6-chlor-4-pyrimidinyl)amino]tetrahydro-2-furanmethanol.

21. (2R-cis)-4-(6-Chlor-9H-purin-9-yl)tetrahydro-2-furanmethanol.

22. (2R-cis)-Tetrahydro-4-(6-methoxy-9H-purin-9-yl)-2-furanmethanol.

23. (2R-cis)-2-Amino-5-nitro-6-[[tetrahydro-5-(hydroxymethyl)-3-furanyl]amino]-4(3H)pyrimidinon.

24. (2R-cis)-2,5-Diamino-6-[[tetrahydro-5-(hydroxymethyl)-3-furanyl]amino]-4(3H)-pyrimidinon.

Europäisches Patentamt

# EUROPÄISCHER RECHERCHENBERICHT

Nummer der Anmeldung

EP 90102731.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | PATENT ABSTRACTS OF JAPAN, unexamined applications, C Sektion, Band 9, Nr. 104, 8. Mai 1985 THE PATENT OFFICE JAPANESE GOVERNMENT, Seite 115 C 279 * Kokai-Nr. 59-231 087 (KYOWA HAKKO KOGYO) * -- | 1 | C 07 D 473/26<br>C 07 D 307/20<br>C 07 D 307/22<br>C 07 D 339/08<br>C 07 D 405/12<br>C 07 D 409/04<br>A 61 K 31/52 |
| A | US - A - 4 289 884 (M.D. BARKER) * Anspruch; Zusammenfassung * -- | 6 | |
| A | EP - A2 - 0 277 599 (ASSAHI GLASS CPT) * Ansprüche; Seiten 3,4 * ---- | 1,18, 19 | |

| RECHERCHIERTE SACHGEBIETE (Int Cl 5) |
|---|
| C 07 D 307/00<br>C 07 D 339/00<br>C 07 D 405/00<br>C 07 D 409/00<br>C 07 D 473/00 |

Der vorliegende Recherchenbericht wurde für alle Patentansprüche erstellt

| Recherchenort | Abschlußdatum der Recherche | Prüfer |
|---|---|---|
| WIEN | 10-05-1990 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82